(19) Europäisches Patentamt
European Patent Office
Office européen des brevets

(11) EP 0 885 922 B1

(12) **EUROPÄISCHE PATENTSCHRIFT**

(45) Veröffentlichungstag und Bekanntmachung des Hinweises auf die Patenterteilung:
**24.11.1999 Patentblatt 1999/47**

(51) Int Cl.6: **C08K 5/07**, C08L 83/04, C09D 183/04

(21) Anmeldenummer: **98109268.7**

(22) Anmeldetag: **22.05.1998**

(54) **Organosiliciumverbindungen enthaltende vernetzbare Zusammensetzungen**

Curable compositions containing organosilicon compounds

Compositions réticulables contenant des composés d'organosilice

(84) Benannte Vertragsstaaten:
**DE FR GB**

(30) Priorität: **05.06.1997 DE 19723669**

(43) Veröffentlichungstag der Anmeldung:
**23.12.1998 Patentblatt 1998/52**

(73) Patentinhaber: **Wacker-Chemie GmbH**
**81737 München (DE)**

(72) Erfinder:
- **Dauth, Jochen, Dr.**
  **84489 Burghausen (DE)**
- **Deubzer, Bernward, Dr.**
  **84489 Burghausen (DE)**
- **Wolferseder, Josef**
  **84367 Tann (DE)**
- **Schnitzer, Klaus**
  **84387 Julbach (DE)**

(74) Vertreter: **Deffner-Lehner, Maria, Dr. et al**
**Wacker-Chemie GmbH,**
**Hanns-Seidel-Platz 4**
**81737 München (DE)**

(56) Entgegenhaltungen:
**US-A- 4 595 739**

- **L. F. MIKHEEVA: "REACTION OF ACETYLENE MONOMAGNESIUM BROMIDE AND ACETYLENE DIMAGNESIUM BROMIDE WITH alpha-DIKETONES" JOURNAL OF ORGANIC CHEMISTRY OF THE USSR. (ZHURNAL ORGANICHESKOI KHIMII), Bd. 1, Nr. 9, 1965, Seiten 1536-1539, XP002077507 NEW YORK US**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die Erfindung betrifft vernetzbare Zusammensetzungen enthaltend

(1) Organosiliciumverbindungen, die Reste mit aliphatischen Kohlenstoff-Kohlenstoff-Mehrfachbindungen aufweisen,
(2) Organosiliciumverbindungen mit Si-gebundenen Wasserstoffatomen oder anstelle von (1) und (2)
(3) Organosiliciumverbindungen, die Reste mit aliphatischen Kohlenstoff-Kohlenstoff-Mehrfachbindungen und Si-gebundene Wasserstoffatome aufweisen,
(4) die Anlagerung von Si-gebundenen Wasserstoff an aliphatische Mehrfachbindung fördernde Katalysatoren und
(5) die Anlagerung von Si-gebundenem Wasserstoff an aliphatische Mehrfachbindung bei Raumtemperatur verzögernde Mittel.

Organopolysiloxanzusammensetzungen, welche durch Reaktion von SiH-Gruppen mit Si-gebundenen olefinischen Gruppen in Gegenwart eines Hydrosilylierungskatalysators härtbar sind, sind beispielsweise aus US-A 2,813,218, US-A 3,249,581 und US-A 3,436,366 bekannt.
Unter dem Begriff Hydrosilylierungskatalysatoren sind dabei die Anlagerung von Si-gebundenen Wasserstoff an aliphatische Mehrfachbindung fördernde Katalysatoren zu verstehen.
Weil das Vernetzen beim Mischen der vorerwähnten Bestandteile (1), (2) bzw. (3) und (4) beginnt, ist es übliche Praxis, additionsvernetzende Organopolysiloxanzusammensetzungen in zwei Komponenten bereitzustellen, von denen die eine das olefinisch ungesättigte Organopolysiloxan und den Hydrosilylierungskatalysator und die andere das Organohydrogenpolysiloxan-Vernetzungsmittel enthält.
Wenn es notwendig ist, die Topfzeit von additionsvernetzenden Organopolysiloxanzusammensetzungen zu verlängern oder eine Einkomponenten-additionsvernetzende Organopolysiloxanzusammensetzung bereitzustellen, kann ein Inhibitor darin enthalten sein.
Unter dem Begriff Inhibitoren sind dabei im Rahmen dieser Erfindung die Anlagerung von Si-gebundenem Wasserstoff an aliphatische Mehrfachbindung bei Raumtemperatur verzögernde Mittel zu verstehen, d.h. Inhibitoren sind Verbindungen, welche additionsvernetzende Organopolysiloxanzusammensetzungen bei Raumtemperatur gegebenenfalls unter Lichtausschluß langsam vernetzen, jedoch das Vernetzen bei erhöhten Temperaturen oder Lichteinwirkung nicht verzögern.
Derartige Inhibitoren sind durch Hitze oder energiereiche Strahlung deaktivierbare Inhibitoren, oder sie sind ausreichend flüchtig, um aus den Organopolysiloxanzusammensetzungen bei erhöhter Temperatur ausgetrieben zu werden.
In der GB-A 1,141,868 (veröffentlicht am 5. Februar 1969, Dow Corning Corporation) sind als Inhibitoren organische Verbindungen mit mindestens einer -C=C-Gruppe, beschrieben. Die Verbindungen zeichnen sich durch ihre hohe Flüchtigkeit und dadurch bedingt durch eine geringe Topfzeit bei leicht erhöhten Temperaturen aus.
Gemäß EP-A 622 420 (offengelegt am 2. November 1994, Rhone-Poulenc Chimie) sind als Inhibitoren Alkinole der allgemeinen Formel R-(R')C(OH)-C=CH mit langen Alkyl- oder Phenylresten beschrieben, die sich durch ihre geringe Flüchtigkeit und dadurch bedingt durch eine ausreichende Topfzeit bei leicht erhöhten Temperaturen auszeichnen.
Inhibitoren mit acetylenischen $\alpha$-Ketogruppen sind in US-A 4,595,739 (ausgegeben am 17. Juni 1986, Rhone-Poulenc Specialites) beschrieben.
Mittels UV-Licht härtbare Siliconzusammensetzungen sind in US-A 4,670,531 (ausgegeben am 2. Juni 1987, General Electric Company) beschrieben, wobei der photolabile Inhibitor durch eine Azogruppe mit einer elektronenziehenden Gruppe in $\alpha$-Stellung gekennzeichnet ist. Diese Inhibitoren besitzen einen geringen molaren Extinktionskoeffizienten und sind zusätzlich thermolabil.
In US-A 5,082,871 (ausgegeben am 21. Januar 1992, General Electric Company) sind Acetylencarbonsäuredialkylester in UV-härtbaren Siliconzusammensetzungen als photolabile Inhibitoren beschrieben. Diese Inhibitoren besitzen ebenfalls einen geringen molaren Extinktionskoeffizienten und eine geringe Photolysegeschwindigkeit.
Es bestand nun die Aufgabe, Inhibitoren bereitzustellen, die nur eine geringe Flüchtigkeit und eine gute Thermostabilität aufweisen, und damit eine ausreichend lange Verzögerung der Vernetzung der additionsvernetzenden Zusammensetzungen auf der Grundlage von Organosiliciumverbindungen bei Raumtemperatur gewährleisten, die aber bei Zufuhr energiereicher Strahlung photolytisch zerfallen und dann eine vollständige Vernetzung der additionsvernetzenden Zusammensetzungen auf der Grundlage von Organosiliciumverbindungen erlauben. Die Aufgabe wird durch die Erfindung gelöst.
Gegenstand der Erfindung sind vernetzbare Zusammensetzungen enthaltend

(1) Organosiliciumverbindungen, die Reste mit aliphatischen Kohlenstoff-Kohlenstoff-Mehrfachbindungen aufweisen,
(2) Organosiliciumverbindungen mit Si-gebundenen Wasserstoffatomen oder anstelle von (1) und (2)
(3) Organosiliciumverbindungen, die Reste mit aliphatischen Kohlenstoff-Kohlenstoff-Mehrfachbindungen und Si-gebundene Wasserstoffatome aufweisen,
(4) die Anlagerung von Si-gebundenen Wasserstoff an aliphatische Mehrfachbindung fördernde Katalysatoren und
(5) die Anlagerung von Si-gebundenem Wasserstoff an aliphatische Mehrfachbindung bei Raumtemperatur verzögernde Mittel der allgemeinen Formel

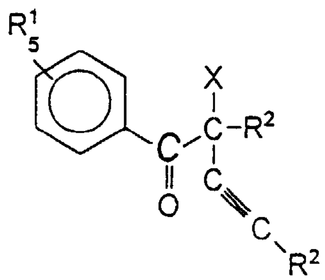

(I)

wobei $R^1$ gleich oder verschieden ist und ein Wasserstoffatom oder einen einwertigen, gesättigten oder ungesättigten gegebenenfalls substituierten Kohlenwasserstoffrest mit 1 bis 12 Kohlenstoffatom(en) je Rest, der durch ein Sauerstoffatom unterbrochen sein kann, oder einen Rest der Formel -CN, -SH, -OH, -Cl, -Br, -OR, -O-C(O)-R, -C(O)OR, -SR, $-NH_2$, -NH-R, -C(O)NHR, -NH-C(O)-R, -COOH bedeutet, wobei R einen einwertigen Kohlenwasserstoffrest mit 1 bis 12 Kohlenstoffatom(en) je Rest bedeutet,
$R^2$ gleich oder verschieden ist und ein Wasserstoffatom oder einen einwertigen, gesättigten oder ungesättigten, gegebenenfalls substituierten Kohlenwasserstoffrest mit 1 bis 12 Kohlenwasserstoffatom(en) je Rest, der durch ein Sauerstoffatom unterbrochen sein kann, bedeutet,

und X ein Rest der Formel -OH, -Cl, -Br und -CN ist, wobei der Rest -OH besonders bevorzugt ist.

Beispiele für Kohlenwasserstoffreste $R^1$ und $R^2$ sind Alkylreste, wie der Methyl-, Ethyl-, n-Propyl-, iso-Propyl-, 1-n-Butyl-, 2-n-Butyl-, iso-Butyl-, tert.-Butyl-, n-Pentyl-, iso-Pentyl-, neo-Pentyl-, tert. Pentylrest; Hexylreste, wie der n-Hexylrest; Heptylreste, wie der n-Heptylrest; Octylreste, wie der n-Octylrest und iso-Octylreste, wie der 2,2,4-Trimethylpentylrest; Nonylreste, wie der n-Nonylrest; Decylreste, wie der n-Decylrest; Dodecylreste, wie der n-Dodecylrest; Cycloalkylreste, wie der Cyclopentyl-, Cyclohexyl-, Cycloheptylreste und Methylcyclohexylreste; Arylreste, wie der Phenyl-, Naphthyl- und Anthryl- und Phenanthrylrest; Alkarylreste, wie o-, m-, p-Tolylreste; Xylylreste und Ethylphenylreste; und Aralkylreste, wie der Benzylrest, der α- und β-Phenylethylrest. Bevorzugt ist ein Wasserstoffatom und der Methylrest.
Beispiele für substituierte Kohlenwasserstoffreste $R^1$ und $R^2$ sind halogenierte Kohlenwasserstoffreste. Beispiele für halogenierte Kohlenwasserstoffreste $R^1$ und $R^2$ sind Halogenalkylreste, wie der 3,3,3-Trifluor-n-propylrest, der 2,2,2,2',2',2'-Hexafluorisopropylrest, der Heptafluorisopropylrest und Halogenarylreste, wie der o-, m- und p-Chlorophenylrest.
Beispiele für Kohlenwasserstoffreste $R^1$ und $R^2$, die durch ein Sauerstoffatom unterbrochen sind, sind der Methoxyethyl- und Ethoxyethylrest.
Beispiele für Kohlenwasserstoffreste R sind Alkylreste, wie der Methyl-, Ethyl-, n-Propyl-, iso-Propyl-, 1-n-Butyl-, 2-n-Butyl-, iso-Butyl-, tert.-Butyl-, n-Pentyl-, iso-Pentyl-, neo-Pentyl-, tert. Pentylrest; Hexylreste, wie der n-Hexylrest; Heptylreste, wie der n-Heptylrest; Octylreste, wie der n-Octylrest und iso-Octylreste, wie der 2,2,4-Trimethylpentylrest; Nonylreste, wie der n-Nonylrest; Decylreste, wie der n-Decylrest; Dodecylreste, wie der n-Dodecylrest; Cycloalkylreste, wie der Cyclopentyl-, Cyclohexyl-, Cycloheptylreste und Methylcyclohexylreste; Arylreste, wie der Phenyl-, Naphthyl- und Anthryl- und Phenanthrylrest; Alkarylreste, wie o-, m-, p-Tolylreste; Xylylreste und Ethylphenylreste; und Aralkylreste, wie der Benzylrest, der α- und β-Phenylethylrest.
Bevorzugte Beispiele für die erfindungsgemäßen Inhibitoren (5) sind Verbindungen der Formel

1)

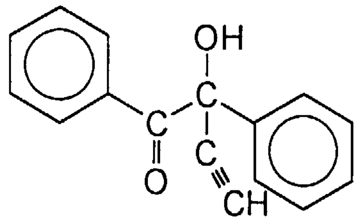

2)

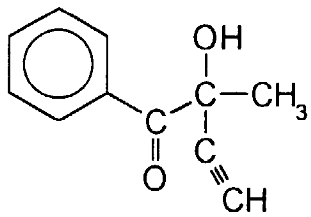

und

3)

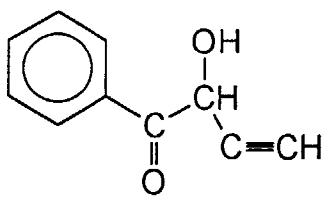

Verbindung 1) ist 1,2-Diphenyl-2-hydroxy-but-3-in-1-on.
Verbindung 2) ist 2-Hydroxy-2-methyl-1-phenyl-but-3-in-1-on.
Verbindung 3) ist 2-Hydroxy-1-phenyl-but-3-in-1-on.
Verbindung 1) ist in Zh. Organ. Khim 1(9), 1536 (1965) von L.F. Mikheeva beschrieben.

Die Herstellung der erfindungsgemäßen Inhibitoren erfolgt nach einem Analogieschluß zu der in Zh. Organ. Khim 1 (9), 1536 (1965) beschriebenen Verfahrensweise.

Die erfindungsgemäßen Inhibitoren der allgemeinen Formel (I) werden vorzugsweise in Mengen von 0,1 Gew.-% bis 1,5 Gew.-%, bevorzugt in Mengen von 0,3 Gew.-% bis 0,7 Gew.-%, bezogen auf das Gesamtgewicht der Organosiliciumverbindungen (1) und (2) bzw. auf das Gesamtgewicht der Organosiliciumverbindung (3), eingesetzt.

Die erfindungsgemäßen Inhibitoren können mit den Organosiliciumverbindungen (1), (2) oder (3) bzw. mit der Katalysatorkomponente (4) im vorab gemischt werden.

Vorzugsweise werden die erfindungsgemäßen Zusammensetzungen mit den Bestandteilen (1), (2) bzw. (3), (4) und (5) in Form von Zweikomponenten-Massen bereitgestellt, wobei die Bestandteile (2) bzw. (3) und (4) von einander getrennt werden.

Die Inhibitoren der Formel (I) können in allen vernetzbaren Zusammensetzungen verwendet werden, in denen auch bisher Inhibitoren verwendet werden konnten, die die Anlagerung von Si-gebundenem Wasserstoff an aliphatische Mehrfachbindung bei Raumtemperatur verzögern.

Als Organopolysiloxane (1), die Reste mit aliphatischen Kohlenstoff-Kohlenstoff-Mehrfachbindungen aufweisen, werden vorzugsweise lineare oder verzweigte Organopolysiloxane aus Einheiten der allgemeinen Formel II

$$R_a^3R_b^4SiO_{\frac{4-a-b}{2}} \qquad \text{(II)},$$

wobei

$R^3$ einen einwertigen, gegebenenfalls substituierten, von aliphatischen Kohlenstoff-Kohlenstoff-Mehrfachbindungen freien Kohlenwasserstoffrest mit 1 bis 18 Kohlenstoffatomen je Rest und
$R^4$ einen einwertigen Kohlenwasserstoffrest mit terminaler, aliphatischer Kohlenstoff-Kohlenstoff-Mehrfachbindung mit 2 bis 8 Kohlenstoffatomen je Rest bedeutet,
a 0, 1, 2 oder 3,
b 0, 1 oder 2
und die Summe a+b 0, 1, 2 oder 3 ist,

mit der Maßgabe, daß durchschnittlich mindestens 2 Reste $R^4$ vorliegen, verwendet.

Bevorzugt als Organosiliciumverbindungen (1) sind Organopolysiloxane der allgemeinen Formel

$$R^4_gR^3_{3-g}SiO(SiR^3_2O)_n(SiR^3R^4O)_mSiR^3_{3-g}R^4_g \qquad \text{(III)}$$

wobei

$R^3$ und $R^4$ die oben dafür angegebene Bedeutung haben,
g 0, 1 oder 2,
n 0 oder eine ganze Zahl von 1 bis 1500 und
m 0 oder eine ganze Zahl von 1 bis 200 ist,

mit der Maßgabe, daß durchschnittlich mindestens 2 Reste $R^4$, enthalten sind.

Im Rahmen dieser Erfindung soll Formel (III) so verstanden werden, daß n Einheiten $-(SiR^3_2O)-$ und m Einheiten $-(SiR^3R^4O)-$in beliebiger Weise im Organopolysiloxanmolekül verteilt sein können.

Als Organosiliciumverbindungen (1) können auch Siloxancopolymere, wie sie in US-A 5,241,034 und in DE-OS 195 22 144 beschrieben sind und die aus Siloxanblöcken und Kohlenwasserstoffblöcken bestehen, verwendet werden.

Die Organopolysiloxane (1) besitzen vorzugsweise eine durchschnittliche Viskosität von 100 bis 10 000 mPa.s bei 25 °C.

Beispiele für Kohlenwasserstoffreste $R^3$ sind Alkylreste, wie der Methyl-, Ethyl-, n-Propyl-, iso-Propyl-, n-Butyl-, iso-Butyl-, tert.- Butyl-, n-Pentyl-, iso-Pentyl-, neo-Pentyl-, tert.-Pentylrest; Hexylreste, wie der n-Hexylrest; Heptylreste, wie der n-Heptylrest; Octylreste, wie der n-Octylrest und iso-Octylreste, wie der 2,2,4-Trimethylpentylrest; Nonylreste, wie der n-Nonylrest; Decylreste, wie der n-Decylrest, Dodecylreste, wie der n-Dodecylrest; Octadecylreste, wie der n-Octadecylrest; Cycloalkylreste, wie Cyclopentyl-, Cyclohexyl-, Cycloheptylreste und Methylcyclohexylreste; Arylreste, wie der Phenyl-, Naphthyl-, Anthryl- und Phenanthrylrest; Alkarylreste, wie o-, m-, p-Tolylreste, Xylylreste und Ethylphenylreste; und Aralkylreste, wie der Benzylrest, der $\alpha$- und der $\beta$-Phenylethylrest.

Beispiele für Reste $R^4$ sind Alkenylreste, wie der Vinyl-, 5-Hexenyl-, Allyl-, 3-Butenyl- und 4-Pentenylrest; und Alkinylreste, wie der Ethinyl-, Propargyl- und 1-Propinrest.

Als Organosiloxane (2), die Si-gebundene Wasserstoffatome aufweisen, werden vorzugsweise lineare, cyclische oder verzweigte Organopolysiloxane aus Einheiten der allgemeinen Formel IV

$$R^3_eH_fSiO_{\frac{4-e-f}{2}} \qquad \text{(IV)},$$

wobei

$R^3$ die oben dafür angegebene Bedeutung hat,
e 0, 1, 2 oder 3,
f 0, 1 oder 2
und die Summe von e+f 0, 1, 2 oder 3 ist,

mit der Maßgabe, daß durchschnittlich mindestens 2 Si-gebundene Wasserstoffatome vorliegen, verwendet.
Bevorzugt werden als Organosiliciumverbindungen (2) Organopolysiloxane der allgemeinen Formel

$$H_hR^3_{3-h}SiO(SiR^3_2O)_o(SiR^3HO)_pSiR^3_{3-h}H_h \qquad (V)$$

wobei

$R^3$ die oben dafür angegebene Bedeutung hat,
h 0, 1 oder 2,
o 0 oder eine ganze Zahl von 1 bis 1500 und
p 0 oder eine ganze Zahl von 1 bis 200 ist,

mit der Maßgabe, daß durchschnittlich mindestens 2 Si-gebundene Wasserstoffatome, vorliegen, verwendet.
Im Rahmen dieser Erfindung soll Formel (V) so verstanden werden, daß o Einheiten $-(SiR^3_2O)-$ und p Einheiten $-(SiR^3HO)-$ in beliebiger Weise im Organopolysiloxanmolekül verteilt sein können.
Die Organopolysiloxane (2) besitzen vorzugsweise eine durchschnittliche Viskosität von 10 bis 1 000 mPa.s bei 25 °C.
Organosiliciumverbindung (2) wird vorzugsweise in Mengen von 0,8 bis 3,0, bevorzugt 1,5 bis 2,5 Grammatom Si-gebundenen Wasserstoff je Mol Si-gebundenen Restes mit aliphatischer Kohlenstoff-Kohlenstoff-Mehrfachbindung in der Organosiliciumverbindung (1) eingesetzt.
Als Organopolysiloxane (3), die aliphatische Kohlenstoff-Kohlenstoff-Mehrfachbindungen und Si-gebundene Wasserstoffatome aufweisen und anstelle von Organopolysiloxanen (1) und (2) verwendet werden können, werden vorzugsweise solche aus Einheiten der Formel

$$R^3_kSiO_{\frac{4-k}{2}} \text{ (VI)}, \qquad R^3_1R^4SiO_{\frac{3-l}{2}} \text{ (VII)} \qquad \text{und } R^3_pHSiO_{\frac{3-d}{2}} \text{ (VIII)},$$

wobei

$R^3$ und $R^4$ die oben dafür angegebene Bedeutung haben,
k 0, 1, 2 oder 3,
1 0, 1 oder 2,
d 0, 1 oder 2 ist,

mit der Maßgabe, daß durchschnittlich mindestens 2 Reste $R^4$ und durchschnittlich mindestens 2 Si-gebundene Wasserstoffatome vorliegen, verwendet.
Beispiele für Organopolysiloxane (3) sind Organopolysiloxane aus $SiO_{4/2}$-, $R^3_3SiO_{1/2}$-, $R^3_2R^4SiO_{1/2}$- und $R^3_2HSiO_{1/2}$-Einheiten, sogenannte MQ-Harze, wobei diese Harze T-Einheiten ($R^3SiO_{3/2}$) und D-Einheiten ($R^4_2SiO$) enthalten können.
Die Organopolysiloxane (3) besitzen vorzugsweise eine durchschnittliche Viskosität von 100 bis 100 000 mPa.s bei 25 °C bzw. sind Feststoffe mit Molekulargewichten von 5 000 bis 50 000 g/mol.
Als die Anlagerung von Si-gebundenem Wasserstoff an aliphatische Mehrfachbindungen fördernde Katalysatoren können auch bei dem erfindungsgemäßen Verfahren die gleichen Katalysatoren eingesetzt werden, die auch bisher zur Förderung der Anlagerung von Si-gebundenem Wasserstoff an aliphatische Mehrfachbindung eingesetzt werden konnten.
Bei den Katalysatoren handelt es sich vorzugsweise um ein Metall aus der Gruppe der Platinmetalle oder um eine Verbindung oder einen Komplex aus der Gruppe Platinmetalle, wie Platin, Palladium oder Rhodium, bevorzugt um eine Verbindung oder einen Komplex des Platins.
Beispiele für solche Katalysatoren sind metallisches und feinverteiltes Platin, das sich auf Trägern wie Siliciumdioxyd, Aluminiumoxyd oder Aktivkohle befinden kann, Verbindungen oder Komplexe von Platin, wie Platinhalogenide, z. B. $PtCl_4$, $H_2PtCl_6*6H_2O$, $Na_2PtCl_4*4H_2O$, Platin-Olefin-Komplexe, Platin-Alkohol-Komplexe, Platin-Ether-Komplexe, Platin-Aldehyd-Komplexe, Platin-Keton-Komplexe, einschließlich Umsetzungsprodukten aus $H_2PtCl_6*6H_2O$ und Cyclohexanon, Platin-Vinylsiloxankomplexe, wie
Platin-1,3-Divinyl-1,1,3,3-tetramethyldisiloxankomplexe mit oder ohne Gehalt an nachweisbaren anorganisch gebundenem Halogen, Bis-(gamma-picolin)-platindichlorid, Trimethylendipyridinplatindichlorid, Dicyclopentadienplatindichlorid, Dimethylsulfoxydethylenplatin-(II)-dichlorid, Cyclooctadien-Platindichlorid, Norbornadien-Platindichlorid,

Gammapicolin-Platindichlorid, Cyclopentadien-Platindichlorid sowie Umsetzungsprodukte von Platintetrachlorid mit Olefin und primärem Amin oder sekundärem Amin oder primärem und sekundärem Amin gemäß US-A 42 92 434, wie das Umsetzungsprodukt aus in 1-Octen gelöstem Platintetrachlorid mit sec.-Butylamin, oder Ammonium-Platinkomplexe gemäß EP-B 1 10 370.

Die Katalysatoren (4) werden vorzugsweise in Mengen von 5 bis 300 Gew.-ppm (Gewichtsteilen je Million Gewichtsteilen), bevorzugt 20 bis 200 Gew.-ppm, jeweils berechnet als elementares Platinmetall und bezogen auf das Gesamtgewicht der Organosiliciumverbindungen (1) und (2) bzw. auf das Gesamtgewicht der Organosiliciumverbindung (3), eingesetzt.

Die erfindungsgemäßen Zusammensetzungen werden vorzugsweise beim Druck der umgebenden Atmosphäre, also etwa bei 1020 hPa (abs.) gehärtet, sie können aber auch bei höheren oder niedrigeren Drücken gehärtet werden.

Die erfindungsgemäßen Zusammensetzungen können unter dem Einfluß energiereicher Strahlung vernetzt werden. Viele Strahlungsarten sind hierbei geeignet, wie etwa Elektronenstrahlen, $\gamma$-Strahlen, Röntgenstrahlen, UV-Licht, wie z.B. solches mit einer Wellenlängen im Bereich von 200 bis 400 nm, und sichtbares Licht, wie z.B. solches mit einer Wellenlänge von 400 bis 600 nm, also sogenanntes "Halogenlicht". Utraviolettlicht kann z.B. in Xenon-, Quecksilbernieder-, Quecksilbermittel- oder Quecksilberhochdrucklampen und Excimerlampen erzeugt werden. Vorzugsweise handelt es sich bei der energiereichen Strahlung, durch welche die erfindungsgemäßen Zusammensetzungen vernetzt werden, um UV-Licht mit einer Wellenlänge im Bereich von 200 bis 400 nm.

Die erfindungsgemäßen Zusammensetzungen können auch ohne Zufuhr von energiereicher Strahlung rein thermisch vernetzt werden, weshalb sie auch nur eine begrenzte Lagerbeständigkeit unter Ausschluß dieser Strahlung aufweisen. Diese sogenannte Topfzeit beträgt aber ein Vielfaches der Vernetzungszeit unter Einfluß von energiereicher Strahlung, weshalb eine rein thermische Härtung zwar möglich, aber nicht bevorzugt ist.

Die erfindungsgemäßen Organopolysiloxanzubereitungen können noch zusätzlich die im Stand der Technik erwähnten Inhibitoren enthalten, obwohl die Mitverwendung nicht bevorzugt ist.

Bei den erfindungsgemäßen Zusammensetzungen können inerte, organische Lösungsmittel mitverwendet werden, obwohl die Mitverwendung von inerten, organischen Lösungsmitteln nicht bevorzugt ist.

Beispiele für inerte, organische Lösungsmittel sind Toluol, Xylol, Isophoron, Octanisomere, n-Butylacetat und Isopropanol.

Des weiteren können die erfindungsgemäßen Massen noch Zusatzstoffe, wie z.B. Füllstoffe, Pigmente und Haftvermittler, wie Silane oder Epoxidverbindungen, enthalten.

Die erfindungsgemäßen Zusammensetzungen können überall dort eingesetzt werden, wo auch bisher vernetzbare Zusammensetzungen auf der Basis von additionshärtenden Siliconmassen eingesetzt wurden. Beispielsweise eignen sich die erfindungsgemäßen Zusammensetzungen hervorragend zur Herstellung von festen Überzügen oder Formkörpern. Beispiele für Oberflächen, auf welche die erfindungsgemäßen Überzüge aufgebracht werden können, sind diejenigen von Papier, Holz, Kork, Kunststoffolien, z.B. Polyethylenfolien oder Polypropylenfolien, keramischen Gegenständen, Glas, einschließlich Glasfasern, Metallen, Pappen, einschließlich solcher aus Asbest, und von gewebtem und ungewebtem Tuch aus natürlichen oder synthetischen organischen Fasern.

Das Auftragen der erfindungsgemäßen Organosiliciummassen auf die zu überziehenden Oberflächen kann in beliebiger, für die Herstellung von Überzügen aus flüssigen Stoffen geeigneter und vielfach bekannter Weise erfolgen, beispielsweise durch Tauchen, Streichen, Gießen, Sprühen, Aufwalzen, Drucken, z.B. mittels einer Offsetgravurüberzugsvorrichtung, Messer- oder Rakelbeschichtung. Insbesondere eignen sich die erfindungsgemäßen Zusammensetzungen zur Verwendung in adhäsiven Beschichtungszusammensetzungen, wie Trennpapierbeschichtungen, Beschichtungen allgemein und in der Elektronik.

In den nachstehend beschriebenen Beispielen beziehen sich alle Angaben von Teilen und Prozentsätzen, falls nicht anders angegeben, auf das Gewicht. Sofern nicht anders angegeben, werden die nachstehenden Beispiele bei einem Druck der umgebenden Atmosphäre, also etwa bei 1000 hPa, und bei Raumtemperatur, also bei etwa 20°C, bzw. bei einer Temperatur, die sich beim Zusammengeben der Reaktanden bei Raumtemperatur ohne zusätzliche Heizung oder Kühlung einstellt, durchgeführt. Im folgenden beziehen sich alle Viskositätsangaben auf eine Temperatur von 25°C.

Beispiel 1:

a) 10,51 g (0,05 mol) Benzil werden in 30 g THF (Tetrahydrofuran) gelöst und unter Rühren auf 0°C abgekühlt. Dazu werden in einem Zeitraum von 45 Minuten 100 ml einer 0,5 molaren Lösung von (0,05 mol) Ethinylmagnesiumchlorid in THF unter Stickstoffatmosphäre zugetropft, so daß die Temperatur nicht über 0°C steigt. Nach 30-minütigem Rühren bei 3°C werden weitere 24 Stunden bei Raumptemperatur gerührt. Danach tropft man 1,8

ml (0,1 mol) Wasser zu und rührt eine weitere Stunde lang. Die Suspension wird mit 10 g (0,084 mol) wasserfreiem Natriumsulfat versetzt, eine Stunde lang gerührt und dann filtriert. Das Filtrat wird bei 40°C im Hochvakuum bis zur Gewichtskonstanz eingeengt. Das braune, klebrige Rohprodukt wird in der Siedehitze in einem Gemisch aus 40 ml n-Hexan und 10 ml Diethylether gelöst, filtriert und langsam kristallisiert. Der Feststoff wird abfiltriert, getrocknet und nochmals nach dem obengenannten Verfahren umkristallisiert. Man erhält 5,1 g (43% d.Th.) 1,2-Diphenyl-2-hydroxy-but-3-in-1-on in Form eines gelben, feinkristallinen Feststoffes (Inhibitor 1).

b) 0,194 g ($8,21.10^{-4}$mol) Inhibitor 1, dessen Herstellung oben unter a) beschrieben wurde, werden in 1 ml Toluol gelöst und dann zu 40 g $\alpha,\omega$-Divinyldimethylpolysiloxan mit einer Viskosität von 500 mPa.s bei 25°C gegeben. Das Lösungsmittel wird bei Raumtemperatur unter reduziertem Druck entfernt. Zur verbleibenden Reaktionsmischung werden 1,05 g eines Mischpolymerisates aus Trimethylsiloxan- und Methylhydrogensiloxaneinheiten mit einer Viskosität von 33 mPa.s bei 25°C, das 1,12 Gew.-% Si-gebundenen Wasserstoff enthält, gegeben. Zum Schluß werden unter Rühren 0,4 g (100 ppm Platin, bezogen auf reines Metall und Gesamtmischung) einer 1 Gew.-%igen Lösung eines DiDivinyltetramethyldisiloxanplatin(O)komplexesin $\alpha,\omega$-Divinyldimethylpolysiloxan mit einer Viskosität von 1000 mPa.s bei 25°C dosiert.

Die Gelzeiten der obengenannten Mischung bei verschiedenen Temperaturen sind in Tabelle 1 zusammengefaßt. Sie wurden mit einem Gel-Timer der Fa. Bachhofer bei einer Füllhöhe von 10 cm bestimmt.

Tabelle 1:

| Temperatur (°C) | Gelzeiten (min) |
|---|---|
| 25 | 2866 |
| 60 | 41 |
| 100 | 4 |

Des weiteren wurde die obengenannte Mischung mit einem Glasstab in ca. 3 µm Schichtdicke auf ein PE-beschichtetes Papier der Fa. PWA Raubling gerakelt und 18 Sekunden lang mit Ultraviolettlicht (UVA - 56 $mW/cm^2$, UVB- 12 $mW/cm^2$) bei 56°C bestrahlt.

Es wurde ein transparente, in organischen Lösungsmitteln unlösliche Beschichtung erhalten, die nicht vernetzte Anteile von weniger als 5 Gew.-% enthielt.

Beispiel 2:

a) 7,41 g (0,05 mol) Phenylpropandion werden in 30 g THF gelöst und unter Rühren auf 0°C abgekühlt. Dazu werden in einem Zeitraum von 30 Minuten 100 ml einer 0,5 molaren Lösung von (0,05 mol) Ethinylmagnesiumchlorid in THF unter Stickstoffatmosphäre zugetropft, so daß die Temperatur nicht über 0°C steigt. Danach wird die Reaktionslösung weitere 48 Stunden bei Raumtemperatur gerührt. Die klare, dunkelbraune Lösung wird jetzt mit 1,8 ml (0,1 mol) Wasser versetzt und eine Stunde bei Raumtemperatur gerührt. Nach dem Trocknen über wasserfreiem Natriumsulfat wird filtriert und das THF destillativ entfernt. Der braune, flüssige Rückstand wird im Hochvakuum bis 180°C fraktionierend destilliert. Der Siedebereich der Hauptfraktion liegt zwischen 153°C und 161 °C. Man erhält 2,4 g (28 % d.Th.) 2-Hydroxy-2-methyl-1-phenyl-but-3-in-1-on in Form einer gelben Flüssigkeit (Inhibitor 2).

b) 0,143 g ($8,22 . 10^{-4}$ mol) Inhibitor 2, dessen Herstellung oben unter a) beschrieben wurde, werden in 1 ml Toluol gelöst und dann zu 40 g $\alpha,\omega$-Divinyldimethylpolysiloxan mit einer Viskosität von 500 mPa.s bei 25°C gegeben. Das Lösungsmittel wird bei Raumtemperatur unter reduziertem Druck entfernt. Zur Reaktionsmischung werden 1,05 g eines Mischpolymerisates aus Trimethylsiloxan- und Methylhydrogensiloxaneinheiten mit einer Viskosität von 33 mPa.s bei 25°C, das 1,12 Gew.-% Si-gebundenden Wasserstoff enthält, gegeben. Zum Schluß werden unter Rühren 0,4 g (100 ppm Platin, bezogen auf reines Metall und Gesamtmischung) einer 1 Gew.-%igen Lösung eines Divinyltetramethyldisiloxanplatin(O)komplexes in $\alpha,\omega$-Divinyldimethylpolysiloxan mit einer Viskosität von 1000 mPa.s bei 25°C dosiert.

Die Gelzeiten der obengenannten Mischung bei verschiedenen Temperaturen sind in Tabelle 2 zusammengefaßt. Sie wurden mit einem Gel-Timer der Fa. Bachhofer bei einer Füllhöhe von 10 cm bestimmt.

Tabelle 2:

| Temperatur (°C) | Gelzeiten (min) |
|---|---|
| 25 | 17 560 |
| 60 | 287 |
| 100 | 6 |

Des weiteren wurde die obengenannte Mischung mit einem Glasstab in ca. 3 µm Schichtdicke auf ein PE-beschichtetes Papier der Fa. PWA Raubling gerakelt und 16 Sekunden lang mit Ultraviolettlicht (UVA - 56 $mW/cm^2$, UVB- 12 $mW/cm^2$) bei 56°C bestrahlt. Es wurde eine transparente, in organischen Lösungsmitteln unlösliche Beschichtung erhalten, die nicht vernetzte Anteile von weniger als 5 Gew.-% enthielt.

## Patentansprüche

1. Vernetzbare Zusammensetzungen enthaltend

(1) Organosiliciumverbindungen, die Reste mit aliphatischen Kohlenstoff-Kohlenstoff-Mehrfachbindungen aufweisen,
(2) Organosiliciumverbindungen mit Si-gebundenen Wasserstoffatomen oder anstelle von (1) und (2)
(3) Organosiliciumverbindungen, die Reste mit aliphatischen Kohlenstoff-Kohlenstoff-Mehrfachbindungen und Si-gebundene Wasserstoffatome aufweisen,
(4) die Anlagerung von Si-gebundenen Wasserstoff an aliphatische Mehrfachbindung fördernde Katalysatoren und
(5) die Anlagerung von Si-gebundenem Wasserstoff an aliphatische Mehrfachbindung bei Raumtemperatur verzögernde Mittel der allgemeinen Formel

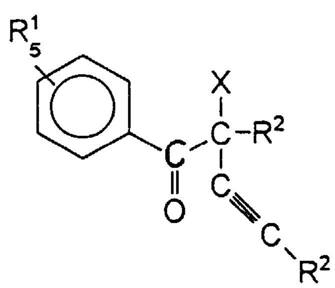

(I)

wobei $R^1$ gleich oder verschieden ist und ein Wasserstoffatom oder einen einwertigen, gesättigten oder ungesättigten gegebenenfalls substituierten Kohlenwasserstoffrest mit 1 bis 12 Kohlenstoffatom(en) je Rest, der durch ein Sauerstoffatom unterbrochen sein kann, oder einen Rest der Formel -CN, -SH, -OH, -Cl, -Br, -OR, -O-C(O)-R, -C(O)OR, -SR, $-NH_2$, -NH-R, -C(O)NHR, -NH-C(O)-R, -COOH bedeutet, wobei R einen einwertigen Kohlenwasserstoffrest mit 1 bis 12 Kohlenstoffatom(en) je Rest bedeutet,
$R^2$ gleich oder verschieden ist und ein Wasserstoffatom oder einen einwertigen, gesättigten oder ungesättigten, gegebenenfalls substituierten Kohlenwasserstoffrest mit 1 bis 12 Kohlenwasserstoffatom(en) je Rest, der durch ein Sauerstoffatom unterbrochen sein kann, bedeutet,

und X ein Rest der Formel -OH, -Cl, -Br und -CN ist.

2. Vernetzbare Zusammensetzungen nach Anspruch 1, dadurch gekennzeichnet, daß X ein Rest der Formel -OH ist.

3. Vernetzbare Zusammensetzungen nach Anspruch 1, dadurch gekennzeichnet, daß als die Anlagerung von Si-gebundenem Wasserstoff an aliphatische Mehrfachbindung bei Raumtemperatur verzögernde Mittel (5) 1,2-Diphenyl-2-hydroxy-but-3-in-1-on verwendet wird.

4. Vernetzbare Zusammensetzungen nach Anspruch 1, dadurch gekennzeichnet, daß als die Anlagerung von Si-gebundenem Wasserstoff an aliphatische Mehrfachbindung bei Raumtemperatur verzögernde Mittel (5) 2-Hydroxy-2-methyl-1-phenyl-but-3-in-1-on verwendet wird.

5. Verfahren zur Herstellung von klebrige Stoffe abweisende Überzüge durch Auftragen von vernetzbaren Zusammensetzungen gemäß einem der Ansprüche 1 bis 4 auf die klebrige Stoffe abweisend zu machenden Oberflächen und anschließende Härtung der Zusammensetzungen.

6. 2-Hydroxy-2-methyl-1-phenyl-but-3-in-1-on.

7. 2-Hydroxy-1-phenyl-but-3-in-1-on.

**Claims**

1. Crosslinkable compositions comprising

(1) organosilicon compounds which contain radicals having aliphatic carbon-carbon multiple bonds,
(2) organosilicon compounds having Si-bonded hydrogen atoms or, in place of (1) and (2),
(3) organosilicon compounds which contain radicals having aliphatic carbon-carbon multiple bonds and Si-bonded hydrogen atoms,
(4) catalysts which promote the addition of Si-bonded hydrogen onto aliphatic multiple bonds and
(5) agents which inhibit the addition of Si-bonded hydrogen onto aliphatic multiple bonds at room temperature, and have the formula

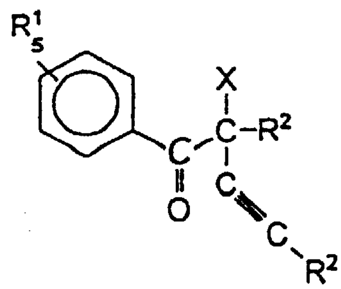

(I)

where $R^1$ are identical or different and are each a hydrogen atom or a monovalent, saturated or unsaturated, substituted or unsubstituted hydrocarbon radical having from 1 to 12 carbon atom(s) per radical, which may be interrupted by an oxygen atom, or a radical of the formula -CN, -SH, -OH, -Cl, -Br, -OR, -O-C(O)-R, -C(O)OR, -SR, $-NH_2$, -NH-R, -C(O)NHR, -NH-C(O)-R, -COOH, where R is a monovalent hydrocarbon radical having from 1 to 12 carbon atom(s) per radical,
$R^2$ are identical or different and are each a hydrogen atom or a monovalent, saturated or unsaturated, substituted or unsubstituted hydrocarbon radical having from 1 to 12 carbon atom(s) per radical, which may be interrupted by an oxygen atom,

and X is a radical of the formula -OH, -Cl, -Br and -CN.

2. Crosslinkable compositions according to Claim 1, characterized in that X is a radical of the formula -OH.

3. Crosslinkable compositions according to Claim 1, characterized in that the agents (5) which inhibit the addition of Si-bonded hydrogen onto aliphatic multiple bonds at room temperature are 1,2-diphenyl-2-hydroxybut-3-yn-1-one.

4. Crosslinkable compositions according to Claim 1, wherein the agents (5) which inhibit the addition of Si-bonded hydrogen onto aliphatic multiple bonds at room temperature are 2-hydroxy-2-methyl-1-phenylbut-3-yn-1-one.

5. Process for producing coatings which repel sticky materials, by applying crosslinkable compositions according to any of Claims 1 to 4 to the surfaces to be made repellant to sticky materials and subsequently curing the compositions.

6. 2-Hydroxy-2-methyl-1-phenylbut-3-yn-1-one.

7. 2-Hydroxy-1-phenylbut-3-yn-1-one.

**Revendications**

1. Compositions réticulables contenant

(1) des composés organosiliciés renfermant des radicaux ayant des liaisons multiples carbone-carbone aliphatiques,
(2) des composés organosiliciés ayant des atomes d'hydrogène liés au silicium ou, à la place de (1) et (2),
(3) des composés organosiliciés renfermant des radicaux ayant des liaisons multiples carbone-carbone aliphatiques et des atomes d'hydrogène liés au silicium,
(4) des catalyseurs favorisant l'addition d'hydrogène lié au silicium sur une liaison multiple aliphatique, et
(5) des agents retardant l'addition d'hydrogène lié au silicium sur une liaison multiple aliphatique à température ambiante, de formule générale

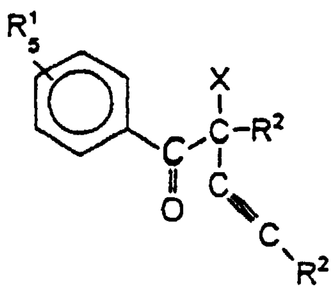

(I)

dans laquelle $R^1$ est identique ou différent et représente un atome d'hydrogène ou un radical hydrocarboné monovalent, saturé ou insaturé, éventuellement substitué ayant de 1 à 12 atome(s) de carbone par radical, qui peut être interrompu par un atome d'oxygène, ou un radical de formule -CN, -SH, -OH, -Cl, -Br, -OR, -O-C(O)-R, -C(O)OR, -SR, $-NH_2$, -NH-R, -C(O)NHR, -NH-C(O)-R, -COOH,R représentant un radical hydrocarboné monovalent ayant de 1 à 12 atome(s) de carbone par radical,
$R^2$ est identique ou différent et représente un atome d'hydrogène ou un radical hydrocarboné monovalent, saturé ou insaturé, éventuellement substitué, ayant de 1 à 12 atome(s) de carbone par radical, qui peut être interrompu par un atome d'oxygène,

et X est un radical de formule -OH, -Cl, -Br et -CN.

2. Compositions réticulables selon la revendication 1, caractérisées en ce que X est un radical de formule -OH.

3. Compositions réticulables selon la revendication 1, caractérisées en ce que l'on utilise la 1,2-diphényl-2-hydroxybut-3-yn-1-one en tant qu'agent (5) retardant l'addition d'hydrogène lié au silicium sur une liaison multiple aliphatique à température ambiante.

4. Compositions réticulables selon la revendication 1, caractérisées en ce que l'on utilise la 2-hydroxy-2-méthyl-1-phénylbut-3-yn-1-one en tant qu'agent (5) retardant l'addition d'hydrogène lié au silicium sur une liaison multiple aliphatique à température ambiante.

5. Procédé de production de revêtements repoussant les substances adhésives par application de compositions réticulables selon l'une quelconque des revendications 1 à 4 sur les surfaces devant être amenées à repousser les substances adhésives, et durcissement consécutif des compositions.

6. 2-hydroxy-2-méthyl-1-phénylbut-3-yn-1-one.

**7.** 2-hydroxy-1-phénylbut-3-yn-1-one.